# NEW EUROPEAN PATENT SPECIFICATION

(11) **EP 0 435 199 B2**
(45) Date of publication and mention of the opposition decision: **19.01.2000**
(45) Mention of the grant of the patent: 29.06.1994
(21) Application number: 90125107.4
(22) Date of filing: 21.12.1990
(51) Int. Cl.: A61L 15/44, A61K 9/70, A61L 15/24

(54) **Acrylic gel material and acrylic gel preparation**
Acrylgel und seine Herstellung
Gel acrylique et sa préparation

(30) Priority: 28.12.1989 JP 34463989; 06.09.1990 JP 23738289
(43) Date of publication of application: 03.07.1991
(73) Proprietor: NITTO DENKO CORPORATION, Osaka (JP)
(72) Inventor: Akemi, Hitoshi, Ibaraki-shi, Osaka (JP); Kinoshita, Takashi, Ibaraki-shi, Osaka (JP); Otsuka, Saburo, Ibaraki-shi, Osaka (JP); Hosaka, Yoshifumi, Ibaraki-shi, Osaka (JP); Nakano, Yoshihisa, Ibaraki-shi, Osaka (JP)
(74) Representative: Grünecker, Kinkeldey, Stockmair & Schwanhäusser Anwaltssozietät

(56) References cited:
- EP-A- 0 062 682
- EP-A- 0 223 524
- EP-A- 0 303 445
- EP-A- 0 309 404
- EP-A- 0 319 988
- WO-A-86/00814
- DE-A- 3 231 400
- DE-A- 3 843 237
- GB-A- 2 086 224
- US-A- 3 222 419
- US-A- 4 374 164
- US-A- 4 608 249

## Description

### FIELD OF THE INVENTION

The present invention relates to an acrylic gel material which is to be applied to the surface of the skin so as to protect the skin surface and an acrylic gel preparation which is used in order to continuously administer a drug component to the living body via the skin surface.

### BACKGROUND OF THE INVENTION

Recently various percutaneous preparations for external use in the form of a preparation to be applied to the skin (for example, plaster, tape), whereby a drug is administered to the living body via the skin surface, have been developed.

Such a percutaneous preparation to be applied to the skin usually involves an adhesive layer having a relatively large adhesiveness in order to secure the fixation of the preparation on the skin surface. Alternately, the whole preparation is covered with a highly adhesive sheet having a large adhesiveness which secures the fixation of the preparation on the skin.

GB-A-2 086 224 discloses a pharmaceutical percutaneous antiphlogistic analgesic adhesive comprising a flexible substrate and an active ingredient (indo-methacin) containing pressure-sensitive adhesive layer closely contacting the flexible substrate.

EP-A-0 303 445 discloses a transdermal patch having a drug reservoir layer on a backing sheet, the drug reservoir layer comprising an adhesive gel base containing an active ingredient (clebopride or a pharmaceutically acceptable salt thereof), the adhesive gel base containing as essential components, a water-soluble polymer, water and a water-holding agent.

Although a percutaneous preparation to be applied to the skin should be surely fixed on the skin so as to secure the migration of a drug component into the skin, an excessively large adhesiveness might bring about a pain or the peeling of the horny substance caused by physical stimulation upon the separation of the preparation from the skin surface. Further, serious skin irritation is sometimes observed.

Thus, the adhesiveness to the skin is an important factor in the development of a percutaneous preparation in practice, and the problem of the skin irritation is also an important factor. Therefore, it has been practically required to develop a preparation which scarcely irritates the skin and can be securely fixed onto the skin.

### SUMMARY OF THE INVENTION

Under these circumstances, the present inventors have conducted extensive studies in order to solve the above problems. As a result, it has been found that a composition comprising an acrylate polymer and a plasticizing liquid ingredient compatible with the acrylate polymer, which plasticizing liquid ingredient is used in an amount larger than a common level, can achieve a softness upon the adhesion to the skin. However, it has been further found that such a composition as described above suffers from a serious decrease in the cohesive power and thus causes cohesion breakage, which makes the peeling of the composition from the skin impossible or brings about skin irritation. Thus such a preparation cannot be used in practice. Furthermore, it has been found that a decrease in the cohesive power of the composition can be prevented and the stress applied to the skin surface upon the separation of a composition can be relieved and dispersed so as to achieve well-balanced skin adhesiveness and skin irritativeness by crosslinking a polymer layer containing an excessive amount of a plasticizing liquid ingredient to thereby form a so-called oily gel, thus completing the present invention.

An object of the present invention is to provide an acrylic gel material to be applied to the skin surface.

Another object of the present invention is to provide an acrylic gel preparation capable of continuously administering a drug component to the living body.

Other objects and effects of the present invention will be apparent from the following description.

The present invention provides an
acrylic gel material comprising a substrate having on one surface thereof a crosslinked gel layer formed by crosslinking a composition comprising an acrylate polymer and a plasticizing liquid ingredient compatible with said acrylate polymer, wherein the weight ratio of said acrylate polymer to said plasticizing liquid ingredient is from 1.0/0.25 to 1.0/2.0 and the thickness of said crosslinked gel layer is from 40 to 150 µm, with the proviso that the crosslinked gel layer is no double layer and that the substrate is no permeable substrate.

The present invention also relates to an acrylic gel preparation wherein a drug component is contained in the above acrylic gel material.

### DETAILED DESCRIPTION OF THE INVENTION

The substrate to be used in the acrylic gel material and the acrylic gel preparation of the present invention is not particularly limited, but materials, which would never suffer from any decrease in the content of the liquid ingredient or the drug contained in the crosslinked gel layer caused by the migration toward another face of the substrate followed by leakage, are preferable. Examples thereof include sole films of polyester, nylon, Saran resins, polyethylene, polypropylene, ethylene-vinyl acetate copolymer, polyvinyl chloride, ethylene-ethyl acrylate copolymer, polytetrafluoroethylene, Surlyn resins and metal foils as well as laminate films comprising these materials. Among these, it is preferred to use a substrate in the form of a laminate film composed of a nonporous sheet comprising one or more materials as cited above and a porous film and to form a crosslinked gel layer on the surface of the porous sheet, to thereby improve the adhesiveness between the substrate and the crosslinked gel layer by the anchoring effect which will be described hereinbelow.

The material of the porous film is not particularly restricted, so long as the anchoring effect to the crosslinked gel layer can be improved. Examples thereof include paper, woven fabric, nonwoven fabric and mechanically perforated sheet. It is particularly preferred to use paper, woven fabric and nonwoven fabric therefor. When the improvement of the anchoring effect and the flexibility of the whole preparation are taken into consideration, the thickness of the porous film is preferably from 10 to 500 µm, and in the case of a thin preparation such as plaster or tape, it is more preferably from 10 to 200 µm. When the laminate film composed of the above porous film and the nonporous sheet is used as the substrate, the thickness of the nonporous sheet is preferably from 0.5 to 50 µm, and more preferably from 1 to 25 µm.

When woven fabric or nonwoven fabric is used as the porous film, the weight per unit area of the woven or nonwoven fabric is preferably from 5 to 30 g/m², still preferably from 8 to 20 g/m², from the viewpoint of the improvement of the anchoring effect.

In the present invention, the crosslinked gel layer to be formed on one surface of the aforesaid substrate is a layer of a crosslinked structure obtained by crosslinking a composition comprising an acrylate polymer and a plasticizing liquid ingredient compatible with the acrylate polymer together with, in the case of a preparation, a drug component, and having an appropriate adhesiveness to the skin and an appropriate cohesive power. The adhesiveness is generally from 70 to 250 g/12 mm width in terms of the adhesiveness to a bakelite plate (the determination method therefor will be described in detail hereinafter) and from 20 to 80 g in the probe-tack test.

The acrylate copolymer serves as a main component constituting the crosslinked gel layer together with the plasticizing liquid ingredient which will be described in detail hereinafter. It sustains a high compatibility with the plasticizing liquid ingredient and thus shows an excellent adhesiveness to the skin surface as well as an excellent shape retention. In the present invention, it is not preferred to use rubber such as natural or synthetic rubber or a silicone polymer since these materials have a poor compatibility with the plasticizing liquid ingredient to be used in the present invention or show a considerably low solubility or release of the drug component. In addition, it is difficult to control the amount of functional groups participating in the crosslinking of such a polymer, compared with the acrylate polymer to be used in the present invention, and thus highly reproducible crosslinking can hardly be achieved. These facts indicate that the above-mentioned polymers are unsuitable in the present invention.

As the acrylate polymer to be used in the present invention, a polymer of an alkyl (meth)acrylate having 4 or more carbon atoms is preferred. It is particularly preferred, from the viewpoint of the convenience in the crosslinking, to use a copolymer obtained by using the aforesaid alkyl (meth)acrylate as the main component.

The terms "(meth)acrylate", etc. used herein mean "acrylate and/or methacrylate", etc.

Examples of the alkyl (meth)acrylate include (meth)acrylates having straight-chain or branched alkyl groups, for example, butyl, pentyl, hexyl, heptyl, octyl, nonnyl, decyl, undecyl, dodecyl and tridecyl. Either one or more of these (meth)acrylates may be used. Among these, 2-ethylhexyl acrylate, isooctyl acrylate and isononyl acrylate are preferably used.

Examples of the monomer to be copolymerized with the aforesaid alkyl (meth)acrylate include monomers containing a carboxyl group such as (meth)acrylic acid, itaconic acid, maleic acid and maleic anhydride; monomers containing a sulfoxyl group such as styrenesulfonic acid, allylsulfonic acid, sulfopropyl (meth)acrylate, (meth)acryloyloxynaphthalenesulfonic acid and acrylamidemethyl propanesulfonic acid; monomers containing a hydroxyl group such as hydroxyethyl (meth)acrylate and hydroxypropyl (meth)acrylate; monomers containing an amide group such as (meth)acrylamide, diacetone acrylamide, dimethyl(meth)acrylamide, N-butylacrylamide, N-methylol(meth)acrylamide and N-methylolpropane(meth)acrylamide; monomers containing an alkylaminoalkyl group such as aminoethyl (meth)acrylate. dimethylaminoethyl (meth)acrylate and tert-butylaminoethyl (meth)acrylate; alkoxyalkyl (meth)acrylates such as methoxyethyl (meth)acrylate and ethoxyethyl (meth)acrylate; (meth)acrylates containing an alkoxy group (or an ether bond in a side chain) such as tetrahydrofurfuryl (meth)acrylate, methoxyethylene glycol (meth)acrylate, methoxydiethylene glycol (meth)acrylate, methoxypolyethylene glycol (meth)acrylate and methoxypolypropylene glycol (meth)acrylate; and vinyl monomers such as (meth)acrylonitrile, vinyl acetate, vinyl propionate, N-vinylpyrrolidone, methyl vinyl pyrrolidone, vinyl pyridine, vinyl piperidine, vinyl pyrimidine, vinyl piperazine, vinyl pyrazine, vinyl pyrol, vinyl imidazole, vinyl caprolactam, vinyl oxazole and vinyl morpholine. Among the above, (meth)acrylic acid, hydroxyethyl (meth)acrylate, 2-methoxyethyl acrylate, acrylamide, N-vinyl-2-pyrrolidone and diacetone acrylamide are preferably used. Either one of these substances or a mixture thereof may be employed in the copolymerization. These copolymerizable monomers may be used in order to control the cohesive power of the gel layer and to improve the solubility of the drug. The amounts of these monomers may be arbitrarily selected depending on the purpose.

Among the above-mentioned acrylate polymers, a copolymer obtained by copolymerizing an alkyl (meth)acrylate with monomer(s) represented by formulae (I) and/or (II):

CH₂ = CHX (I)

CH₂ = CRY (II)

(wherein R represents a hydrogen atom or a methyl group; X represents a group having at least one nitrogen atom or a nitrogen atom and an oxygen atom; Y represents a hydrogen atom or -COOR'; and R' represents a group having at least one nitrogen atom or a nitrogen atom and an oxygen atom, or a hydroxy lower alkyl group) may preferably be used in the present invention. By copolymerizing the monomers represented by formulae (I) and (II), the extent of crosslinking and the properties of the obtained gel can be controlled. The weight ratio of the (meth)acrylate to the monomer(s) of formulae (I) and/or (II) is preferably (40-99)/(1-60), more preferably (60-98)/(2-40) while the total amount of these monomers being 100.

The plasticizing liquid ingredient to be used in the present invention has a high compatibility with the above-mentioned acrylate copolymer. The plasticizing liquid ingredient moderately plasticizes the crosslinked gel layer and thus imparts a flexible texture, to thereby relive a pain or skin irritativeness caused by the skin adhesiveness upon the separation of the crosslinked gel layer from the skin surface.

Therefore the plasticizing liquid ingredient is selected from among materials having a plasticizing effect. A substance which further has an absorption-promoting effect may be preferably selected therefor to thereby improve the percutaneous absorption of the drug component used together.

Examples of the plasticizing liquid ingredient include glycols such as ethylene glycol, diethylyene glycol, triethylene glycol, propylene glycol, polyethylene glycol and polypropylene glycol; fats and oils such as olive oil, castor oil, squalene and lanolin; organic solvents such as dimethyl decyl sulfoxide, methyl acetyl sulfoxide, dimethyl sulfoxide, dimethylformamide, dimethylacetamide, dimethyllaurylamide, dodecyl pyrrolidone and isosorbitol; liquid surfactants; plasticizers such as diisopropyl adipate, phthalates and diethyl sebacate; hydrocarbons such as liquid paraffin; ethoxylated stearyl alcohol, glycerol esters, isopropyl myristate, isotridecyl myristate, ethyl laurate, N-methylpyrrolidone, ethyl oleate, oleic acid, isopropyl adipate, isopropyl palmitate, octyl palmitate and 1,3-butanediol. Among the above, phthalic acid esters, isopropyl myristate, isotridecyl myristate and octyl palmitate are preferably used. Either one of these substances or a mixture thereof may be used.

The aforesaid acrylate polymer and the aforesaid plasticizing liquid ingredient is contained in the crosslinked gel layer at a weight ratio of from 1.0/0.25 to 1.0/2.0, preferably from 1.0/0.4 to 1.0/2.0 and more preferably from 1.0/0.6 to 1.0/1.8, from the viewpoint of reducing the skin irritativeness. Namely, it is preferred to use a considerably large amount of the plasticizing liquid ingredient. In contrast, a conventional preparation usually contains a plasticizing liquid ingredient at a weight ratio less than 1.0/0.25. Such a low content of the plasticizing liquid ingredient would sometimes make it impossible to achieve a satisfactory low level of the skin irritativeness, from a practical viewpoint.

In the present invention, the composition thus-obtained is then crosslinked by an appropriate crosslinking procedure so as to give a gel, thus preventing the leakage of the plasticizing liquid ingredient contained in the preparation and imparting a cohesive power, as described above. The crosslinking may be effected by a physical means such as irradiation (for example, UV irradiation or electron beam irradiation) or a chemical means with the use of a crosslinking agent (for example, polyisocyanate compound, organic peroxide, organic metal salt, metal alcoholate, metal chelate compound, polyfunctional compound).

Among these crosslinking procedures, irradiation or the use of an organic peroxide might induce decomposition in the cases of some drug component. Further, the use of a highly reactive isocyanate or a metal salt or an organic metal salt commonly used in crosslinking reactions might sometimes cause an increase in the viscosity of the solution, which lowers the workability thereof. It is also possible to preliminarily copolymerize a polyfunctional monomer such as diacrylate with the acrylate polymer. In this case, however, there is a possibility that the viscosity of the solution would increase at the polymerization.

In the present invention, therefore, it is preferred to select trifunctional isocyanate or a metal alcoholate or a metal chelate compound comprising titanium or aluminum from among the aforesaid crosslinking agents, from the viewpoints of reactivity and handling. These crosslinking agents would not cause any increase in the viscosity of the solution until the completion of the application and drying, which means that they are excellent in workability. When these crosslinking agents are used, the crosslinking reaction can be effected to a certain extent by coating and drying the gel layer, but the coated and dried gel layer is preferably aged at from 40 to 70 °C for stabilizing the properties of the gel layer. The aging time varies depending on the addition amount and the kind of the functional groups of the crosslinking agent, and is generally from 2 to 3 days. Such a crosslinking agent is preferably used in an amount of from 0.01 to 2.0 parts by weight per 100 parts by weight of the acrylate polymer. When the acrylate polymer has no such a functional group as to react with the above-mentioned crosslinking agent, the material to be crosslinked may be modified by, for example, treating with an alkali to thereby enable the crosslinking.

In the present invention, a drug component may be added to the crosslinked gel layer thus-obtained to thereby give a gel preparation. The drug component to be added may be arbitrarily selected depending on the purpose of the treatment. Namely, any drug component may be used so long as it can be percutaneously absorbed and examples thereof include cortisteroid, analgesic/antiinflammatory agent, hypnotic/sedative agent, ataraxic, antihypertensive agent, hypotensive diuretic, antibiotic, anesthetic, antibacterial agent, fungicide, vitamin preparation, coronary dilator (except isosorbide dinitrate), antihistamine, antitussive agent, sex hormone (except estrogen), antidepressant, cerebral circulatory improver, antivommiting agent. antitumor agent and biogenic. Either one of these drugs or a mixture thereof may be used. In order to achieve the uniform dispersion in the crosslinked gel layer and to promote percutaneous absorption, it is preferred to use a hydrophobic drug (having a solubility of 0.4 g/100 ml of water or less at room temperature) from among the aforesaid drug component.

The content of the drug component may be appropriately determined depending on the selected drug component and the purpose of the administration. It is generally contained in the crosslinked gel layer in an amount of from 1 to 40% by weight, preferably from 3 to 30% by weight. When the content of the drug is smaller than 1% by weight, the release of a therapeutically effective amount of the drug cannot be expected. When it exceeds 40% by weight, on the other hand, no improvement in the effect cannot be achieved any more. Thus, both of these cases bring about economical disadvantages.

The method for preparing the gel material and the gel preparation according to the present invention is not particularly limited. For example, a drug solution is added to a solution of an acrylate polymer followed by stirring, and a plasticizing liquid ingredient is added thereto to form a uniform solution. A crosslinking agent in the form of a solution is added to the above-ontained solution and the viscosity of the resulting solution is adjusted by ethyl acetate to prepare a gel layer coating composition. The coating composition is coated on a separator, and then dried to form a gel layer. The thickness of the gel layer after drying is from 40 to 150 µm. The resulting gel layer is transferred to a substrate, and then, if necessary, aged at from 40 to 70 °C to obtain a gel preparation according to the present invention. A gel material according to the present invention can be prepared in the same manner as above except that the drug solution is not used.

When the drug component is to be added to the acrylic gel preparation of the present invention, it is preferred that the crosslinked gel layer contains the drug as described above. Alternately, it is possible that the drug component is not contained in the crosslinked gel layer but dissolved in an appropriate solvent and the solution thus-obtained is located at the interface between the crosslinked gel layer and the substrate followed by sealing the periphery of the preparation. When the layer containing the drug component is separated from the crosslinked gel layer in such a manner as described above, the decomposition of the drug component upon storage can be suppressed in the case of a drug component which is liable to undergo decomposition. In this case, the release of the drug component can be severely controlled by locating a microporous film between the layer containing the drug component and the crosslinked gel layer.

Each of the acrylic gel preparation and the acrylic gel preparation of the present invention, which has the aforesaid structure, comprises a crosslinked gel layer containing a large amount of the plasticizing liquid ingredient compatible with the acrylate polymer. Thus, it is possible to impart a flexibility to the crosslinked gel layer and to reduce the skin irritativeness while maintaining the cohesive power of the gel layer. When the preparation of the present invention is to be separated from the surface of the skin, therefore, the pain and skin irritativeness caused by the adhesiveness can be reduced. Thus, the acrylic gel preparation of the present invention has well-balanced adhesiveness to the skin and nonirritativeness. Further, the acrylic gel preparation containing a drug component can appropriately release the drug component onto the surface of the skin. Thus, it is useful for preventing and treating various diseases through the percutaneous administration of the drug component.

In the present invention, the standard of the painless removal of the gel preparation from the surface of the skin is specified as follows. In a peeling test with the use of volunteers, namely, the amount of the peeled horny substance caused by the removal of the preparation of the present invention corresponds to 1/5 to 2/3 of those observed in the case of control preparations free from any plasticizing liquid ingredient. When the amount of the peeled horny substance is outside the above range, either a pain or an insufficient skin-adhesion might be caused.

The present invention will be described in more detail by referring to the following Examples and Comparative Examples, but the present invention is not construed as being limited thereto. In the following Examples and Comparative Examples, all parts and percents are by weight.

### EXAMPLE 1

95 Parts of 2-ethylhexyl acrylate and 5 parts of acrylic acid were copolymerized in ethyl acetate under an inert gas atmosphere to thereby give an acrylate polymer solution.

To 50 parts of the solid content of the above solution, 50 parts of isopropyl myristate was added. To 99.8 parts of the above acrylic polymer, 0.2 parts of aluminum tris(acetylacetonate) which was in the form of a 10% solution in acetylacetone was added. Further, ethyl acetate was added thereto so as to adjust the viscosity of the mixture.

The viscous solution thus-obtained was applied to a polyester separator (thickness: 75 µm) in such a manner as to give a thickness of 80 µm after drying. After drying and crosslinking, a crosslinked gel layer was formed.

The crosslinked gel layer thus-obtained was adhered to the nonwoven fabric face of a laminate film (i.e., a substrate), which was obtained by extruding polyester having a thickness of 2 µm on a polyester nonwoven fabric (12 g/m²). Thus, an acrylic gel material of the present invention was obtained.

### EXAMPLE 2

The procedure of Example 1 was repeated except that 45 parts of isopropyl myristate and 10 parts ketoprofen were added to 45 parts of the solid content of the acrylate polymer obtained in Example 1. Thus, an acrylic gel preparation of the present invention was obtained.

### EXAMPLE 3

The procedure of Example 1 was repeated except that the isopropyl myristate was replaced by octyl palmitate. Thus, an acrylic gel preparation of the present invention was obtained.

### COMPARATIVE EXAMPLE 1

The procedure of Example 1 was repeated except that the acrylate polymer prepared in Example 1 was not subjected to the crosslinking but used as such. Thus, an acrylic gel material containing the plasticizing liquid ingredient was obtained.

This acrylic gel material was broken because of low cohesion power. Thus, it could be subjected to none of the tests which will be described hereinafter.

### Comparative Example 2

The procedure of Example 1 was repeated except that 10 parts of ketoprofen was added to 90 parts of the solid content of the acrylate polymer solution prepared in Example 1 followed by adding ethyl acetate to thereby adjust the viscosity. Thus, an uncrosslinked acrylic preparation free from any plasticizing liquid ingredient was obtained.

### COMPARATIVE EXAMPLE 3

The procedure of Comparative Example 2 was repeated except that 0.2 part of a crosslinking agent (aluminum tris(acetylacetonate)) was added to the polymer solid content. Thus, a crosslinked acrylate preparation free from any plasticizing liquid ingredient was obtained.

### EXAMPLE 4

75 Parts of 2-ethylhexyl acrylate, 23 parts of N-vinyl-2-pyrrolidone and 2 parts of acrylic acid were copolymerized in ethyl acetate under an inert gas atmosphere to thereby give an acrylate polymer solution.

To 50 parts of the solid content of the above solution, 50 parts of octyl palmitate was added. To 99.8 parts of the above acrylic polymer, 0.2 parts of ethylacetoacetate aluminum diisopropylate which was in the form of a 10% solution in acetylacetone was added. Further, ethyl acetate was added thereto so as to adjust the viscosity of the mixture.

Next, the obtained viscous solution was treated in the same manner as in Example 1 to thereby give an acrylic gel material of the present invention.

### EXAMPLE 5

The procedure of Example 4 was repeated except that the 40 parts of octyl palmitate and 15 parts of nifedipine were added to 45 parts of the solid content of the acrylate polymer prepared in Example 4. Thus, a crosslinked gel layer was formed.

Next, the same procedure as in Example 1 was performed except that the polyester film used as a substrate in Example 1 on which aluminum is vapor-deposited for light shielding. Thus, an acrylic gel preparation of the present invention was obtained.

### EXAMPLE 6

The procedure of Example 5 was repeated except that the octyl palmitate was replaced with isotridecyl myristate. Thus, an acrylic gel preparation of the present invention was obtained.

### COMPARATIVE EXAMPLE 4

The procedure of Example 4 was repeated except that the acrylate polymer prepared in Example 4 was not subjected to the crosslinking but used as such. Thus, an uncrosslinked acrylic gel plaster free from any plasticizing liquid ingredient was obtained.

This acrylic gel plaster was broken because of low cohesive power. Thus, it could be subjected to none of the tests which will be described hereinafter.

### COMPARATIVE EXAMPLE 5

The procedure of Example 4 was repeated except that 15 parts of nifedipine was added to 85 parts of the solid content of the acrylate polymer solution prepared in Example 4 followed by adding ethyl acetate to thereby adjust the viscosity. Thus an uncrosslinked acrylic preparation free from any plasticizing liquid ingredient was obtained.

### COMPARATIVE EXAMPLE 6

The procedure of Comparative Example 5 was repeated except that 0.2 part of a crosslinking agent (ethylacetoacetate aluminum diisopropylate) was added to the polymer solid content. Thus, a crosslinked acrylate preparation free from any plasticizing liquid ingredient was obtained.

### COMPARATIVE EXAMPLE 7

The procedure of Comparative Example 4 was repeated except that the acrylate polymer used in Comparative Example 4 was replaced with a polyisobutyrene rubber polymer comprising 10 parts of polyisobutyrene (viscosity-average molecular weight: 990,000), 15 parts of polyisobutyrene (viscosity-average molecular weight: 60,000), 3 parts of polyisobutyrene (viscosity-average molecular weight: 1260) and 7 parts of an alicyclic petroleum resin (softening point: 100 °C) while the ethyl acetate was replaced with toluene. Thus, a rubber gel preparation was obtained.

This gel preparation showed the precipitation of a large amount of nifedipine immediately after the completion of the preparation.

### EXAMPLE 7

70 Parts of 2-ethylhexyl acrylate, 25 parts vinyl acetate and 5 parts of 2-hydroxyethyl methacrylate were copolymerized in ethyl acetate under an inert gas atmosphere to thereby give an acrylate polymer solution.

To 50 parts of the solid content of the above solution, 50 parts of isotridecyl myristate was added. To 99.7 parts of the above acrylic polymer, 0.3 parts of trifunctional isocyanate ("Coronate HL" manufactured by Nippon Polyurethane Co., Ltd.) in the form of a 10% solution in ethyl acetate was added. Further, ethyl acetate was added thereto so as to adjust the viscosity.

The viscous solution thus-obtained was treated in the same manner as in Example 1 to thereby give an acrylic gel material of the present invention.

### EXAMPLE 8

The procedure of Example 7 was repeated except that 45 parts of tridecyl myristate and 10 parts of clonidine were added to 45 parts of the solid content of the acrylate polymer prepared in Example 7 and that 0.3 part of trifunctional isocyanate ("Coronate HL" manufactured by Nippon Polyurethane Co., Ltd.) in the form of a 10% solution in ethyl acetate was added to 99.7 parts of the acrylate polymer. Thus, a crosslinked gel layer was formed. Next, the crosslinked gel layer was adhered to the same substrate as the one used in Example 1 to thereby give an acrylic gel preparation of the present invention.

### EXAMPLE 9

The procedure of Example 8 was repeated except that the isotridecyl myristate was replaced with isopropyl myristate. Thus, an acrylic gel preparation of the present invention was obtained.

### COMPARATIVE EXAMPLE 8

The procedure of Example 7 was repeated except that the no crosslinking agent was added. Thus, an uncrosslinked acrylic gel material containing a plasticizing liquid ingredient was obtained.

This acrylic gel material was broken because of low cohesive power. Thus, it could be subjected to none of the tests which will be described hereinafter.

### COMPARATIVE EXAMPLE 9

The procedure of Example 7 was repeated except that 0.3 part of trifunctional isocyanate ("Coronate HL" manufactured by Nippon Polyurethane Co., Ltd.) in the form of a 10% solution in ethyl acetate was added to 99.7 part of the solid content of the acrylate polymer solution prepared in Example 7 followed by adjusting the viscosity with ethyl acetate. Thus, a crosslinked acrylic plaster free from any plasticizing liquid ingredient was obtained.

### COMPARATIVE EXAMPLE 10

The procedure of Example 7 was repeated except that 10 parts of clonidine was added to 90 parts of the solid content of the acrylate polymer solution prepared in Example 7 and that 0.3 part of trifunctional isocyanate ("Coronate HL" manufactured by Nippon Polyurethane Co., Ltd.) in the form of a 10% solution in ethyl acetate was added to 99.7 part of the solid content of the acrylate polymer solution prepared in Example 7 followed by adjusting the viscosity with ethyl acetate. Thus, a crosslinked acrylic preparation free from any plasticizing liquid ingredient was obtained.

### COMPARATIVE EXAMPLE 11

By using the acrylate polymer solution prepared in Example 1, a plaster containing neither any plasticizing liquid ingredient, crosslinking agent nor drug component was prepared. The same substrate as the one used in Example 1 was employed.

### COMPARATIVE EXAMPLE 12

The procedure of Example 1 was repeated except that 18 parts of isopropyl myristate was added to 82 part of the solid content of the acrylate polymer solution. Thus, an acrylic gel material was obtained.

### TEST EXAMPLE

Each of the gel materials and gel preparations obtained in the above Examples and Comparative Examples was stored at 40 °C under a relative humidity of 75% for 2 weeks. Next, these samples were subjected to the following tests. In the determination of the peeled horny substance, samples comprising a single-layer film (thickness: 9 µm) having no nonwoven fabric laminated as the substrate were employed, since the absorption of the dyeing solution by the nonwoven fabric in the substrate might substantially lower the accuracy of the determination. Further, the preparations containing clonidine were not subjected to the human patch test. Tables 1 and 2 show the results.

### Rabbit patch test

Each of the samples obtained in Examples and Comparative Examples was applied onto the dorsal part of a rabbit from which the hair had been removed. Then, 2 ml portions of the blood of the rabbit were collected after 1.0, 2.5, 4.0, 6.0 and 8.0 hours and the concentration of isosorbide dinitrate in each blood sample was determined by gas chromatography. The samples containing clonidine were cut into a piece of 3 cm² (1.73 cm x 1.73 cm) while the others were cut into a piece of 50 cm² (7.1 cm x 7.1 cm).

### Adhesion test

Each sample in the form of a strip of 12 mm in width was applied to a bakelite plate. Then, a roller of a load of 300 g was moved thereon back and force to thereby secure the adhesion of the sample to the plate. Subsequently, the sample was peeled off in the direction of 180° at a rate of 300 mm/min and the peeling force upon this procedure was measured.

### Tack test

The tack of each sample was evaluated by the probe tack method with the use of a rheometer.

The sample was fixed on a metal plate in such a manner that the face to be adhered to the skin was placed upward. Then, a spherical probe (diameter: 10 mm) was contacted with the sample under a load of 100 g at a rate of 2 cm/min. After maintaining this state for 20 seconds, the spherical probe was separated therefrom at the same rate. The peeling force upon this procedure was measured.

### Pain at peeling

Samples were applied to the inside of upper arms of 5 volunteers. After 30 minutes, the samples were peeled off and the pain thus caused was examined. The pain was evaluated in five grades (1: the least pain) and expressed in the mean of the volunteers. As a standard, the sample prepared in Comparative Example 1 was referred to as 5.

### Peeled horny substance

Circular samples (diameter: 16 mm) were applied to the inside of upper arms of 3 volunteers (A, B and C). After 30 minutes, the samples were peeled off and immersed in a dyeing solution composed of 0.5% of Gentian violet. 0.5% of Brillian green end 98.5% of distilled water for 3 minutes followed by washing with water to thereby dye horny cells.

These samples were then immersed in a 5% aqueous solution of sodium dodecyl sulfate over day and night to thereby extract the dyeing solution. Then, the absorbance of the extract was measured at 595 nm to thereby compare the number of the peeled horny cells. That is, it was considered that a higher absorbance would indicate the larger amount of the peeled horny substance.

A good correlation was observed between the number of the peeled horny cells counted under a stereoscopic microscope and the above-mentioned absorbance.

**TABLE 1**

| | Rabbit patch test | |
|---|---|---|
| | Maximum blood level (ng/ml) | Time required for achieving maximum blood level (hour) |
| Example 2 | 2,970 | 2.0 |
| Example 3 | 2,840 | 2.0 |
| Example 5 | 205 | 4.0 |
| Example 6 | 198 | 4.0 |
| Example 8 | 16 | 6.0 |
| Example 9 | 20 | 6.0 |
| Comparative Example 2 | 1,840 | 2.0 |
| Comparative Example 3 | 1,510 | 2.0 |
| Comparative Example 5 | 58 | 4.0 |
| Comparative Example 6 | 49 | 4.0 |
| Comparative Example 7 | 11 | 4.0 |
| Comparative Example 10 | 8 | 6.0 |

**TABLE 2**

| | Adhesiveness (g) | Tack (g) | Pain | Peeled horny substance (x 10⁻²) | | |
|---|---|---|---|---|---|---|
| | | | | A | B | C |
| Example 1 | 184 | 45 | 1.6 | 43.4 | 44.4 | 27.4 |
| Example 2 | 176 | 43 | 1.6 | 48.7 | 40.1 | 33.8 |
| Example 3 | 181 | 44 | 1.8 | 42.2 | 39.5 | 34.1 |
| Example 4 | 156 | 38 | 1.4 | 35.1 | 38.6 | 29.9 |
| Example 5 | 142 | 34 | 1.4 | 37.2 | 36.6 | 35.5 |
| Example 6 | 180 | 44 | 1.6 | 49.1 | 45.5 | 28.7 |
| Example 7 | 151 | 36 | 1.2 | 36.1 | 37.2 | 31.1 |
| Example 8 | 154 | 37 | - | - | - | - |
| Example 9 | 132 | 32 | - | - | - | - |
| Comparative Example 2 | 532 | 129 | 5.0 | 148 | 162 | 131 |
| Comparative Example 3 | 516 | 125 | 5.0 | 162 | 181 | 141 |
| Comparative Example 5 | 451 | 109 | 4.6 | 159 | 132 | 124 |
| Comparative Example 6 | 484 | 117 | 4.8 | 179 | 132 | 138 |
| Comparative Example 7 | 1,187 | 282 | 5.0 | 140 | 70.1 | 72.2 |
| Comparative Example 9 | 421 | 102 | 4.6 | 141 | 125 | 119 |
| Comparative Example 10 | 432 | 105 | - | - | - | - |
| Comparative Example 11 | 529 | 138 | 5.0 | 151 | 192 | 138 |
| Comparative Example 12 | 531 | 140 | 4.6 | 141 | 106 | 122 |

As the above Tables 1 and 2 clearly shows, the acrylic gel material and the acrylic gel preparation of the present invention showed less pain at the peeling and, furthermore, suffered from the peeling of a smaller amount of the horny substance, compared with the products of Comparative Examples. In the case of the gel preparation containing the drug component, furthermore, a large amount of the drug would be rapidly absorbed percutaneously.

While the invention has been described in detail and with reference to specific examples thereof, it will be apparent to one skilled in the art that various changes and modifications can be made therein without departing from the spirit and scope thereof.

## Claims (Claims for the following Contracting State(s): CH, DE, FR, GB, IT, LI, NL)

1. An acrylic gel material comprising a substrate having on one surface thereof a crosslinked gel layer formed by crosslinking a composition comprising an acrylate polymer and a plasticizing liquid ingredient compatible with said acrylate polymer, wherein the weight ratio of said acrylate polymer to said plasticizing liquid ingredient is from 1.0/0.25 to 1.0/2.0 and the thickness of said crosslinked gel layer is from 40 to 150 µm, with the proviso that the crosslinked gel layer is no double layer and that the substrate is no permeable substrate.

2. An acrylic gel material as claimed in claim 1, wherein the weight ratio of said acrylate polymer to said plasticizing liquid ingredient is from 1.0/0.4 to 1.0/2.0.

3. An acrylic gel material as claimed in claim 2, wherein the weight ratio of said acrylate polymer to said plasticizing liquid ingredient is from 1.0/0.6 to 1.0/1.8.

4. An acrylic gel material as claimed in claim 1, wherein the crosslinking is effected by using a crosslinking agent selected from a metal alcoholate and a metal chelate each comprising titanium or aluminum, and trifunctional isocyanate.

5. An acrylic gel preparation wherein a drug component is contained in an acrylic gel material as claimed in claim 1.

6. An acrylic gel preparation as claimed in claim 5, wherein the content of said drug component is from 1 to 40% by weight based on the total amount of said crosslinked gel layer.

7. An acrylic gel preparation as claimed in claim 6, wherein the content of said drug component is from 3 to 30% by weight based on the total amount of said crosslinked gel layer.

## Claims (Claims for the following Contracting State(s): ES)

1. A method for preparing an acrylic gel material by crosslinking a composition comprising an acrylate polymer and a plasticizing liquid ingredient compatible with said acrylate polymer to form a crosslinked gel layer provided on one surface of a substrate, wherein the weight ratio of said acrylate polymer to said plasticizing liquid ingredient is from 1.0/0.25 to 1.0/2.0 and the thickness of said crosslinked gel layer is from 40 to 150 µm, with the proviso that the crosslinked gel layer is no double layer and that the substrate is no permeable substrate.

2. The method of claim 1, wherein the weight ratio of said acrylate polymer to said plasticizing liquid ingredient is from 1.0/0.4 to 1.0/2.0.

3. The method of claim 2, wherein the weight ratio of said acrylate polymer to said plasticizing liquid ingredient is from 1.0/0.6 to 1.0/1.8.

4. The method of claim 1, wherein said composition is crosslinked by using a crosslinking agent selected from a metal alcoholate and a metal chelate each comprising titanium or aluminum, and trifunctional isocyanate.

5. A method for preparing an acrylic gel preparation by adding a drug component to the acrylic gel material as claimed in claim 1.

6. The method of claim 5, wherein the content of said drug component is from 1 to 40% by weight based on the total amount of said crosslinked gel layer.

7. The method of claim 6, wherein the content of said drug component is from 3 to 30% by weight based on the total amount of said crosslinked gel layer.

## Patentansprüche (Patentansprüche für folgende(n) Vertragsstaat(en): CH, DE, FR, GB, IT, LI, NL)

1. Acrylgelmaterial, das ein Substrat mit einer vernetzten Gelschicht auf einer Oberfläche desselben umfaßt, welche durch Vernetzen einer Zusammensetzung gebildet wurde, die ein Acrylatpolymer und einen mit dem Acrylatpolymer verträglichen, weichmachenden, flüssigen Bestandteil umfaßt, wobei das Gewichtsverhältnis des Acrylatpolymers zu dem weichmachenden, flüssigen Bestandteil 1,0/0,25 bis 1,0/2,0 beträgt und die Dicke der vernetzten Gelschicht 40 bis 150 µm beträgt, mit der Maßgabe, daß die vernetzte Gelschicht keine Doppelschicht ist und daß das Substrat kein durchlässiges Substrat ist.

2. Acrylgelmaterial wie in Anspruch 1 beansprucht, bei dem das Gewichtsverhältnis des Acrylatpolymers zu dem weichmachenden, flüssigen Bestandteil 1,0/0,4 bis 1,0/2,0 beträgt.

3. Acrylgelmaterial wie in Anspruch 2 beansprucht, bei dem das Gewichtsverhältnis des Acrylatpolymers zu dem weichmachenden, flüssigen Bestandteil 1,0/0,6 bis 1,0/1,8 beträgt.

4. Acrylgelmaterial wie in Anspruch 1 beansprucht, bei dem die Vernetzung durch Verwenden eines Vernetzungsmittels bewirkt wird, das aus einem Metallalkoholat und einem Metallchelat, die jeweils Titan oder Aluminium umfassen, und einem trifunktionellen Isocyanat ausgewählt ist.

5. Acrylgelzubereitung, bei der ein Arzneimittelbestandteil in einem in Anspruch 1 beanspruchten Acrylgelmaterial enthalten ist.

6. Acrylgelzubereitung wie in Anspruch 5 beansprucht, bei der der Gehalt des Arzneimittelbestandteils von 1 bis 40 Gew.-% bezogen auf die Gesamtmenge der vernetzten Gelschicht beträgt.

7. Acrylgelzubereitung wie in Anspruch 6 beansprucht, bei der der Gehalt des Arzneimittelbestandteils von 3 bis 30 Gew.-% bezogen auf die Gesamtmenge der vernetzten Gelschicht beträgt.

## Patentansprüche (Patentansprüche für folgende(n) Vertragsstaat(en): ES)

1. Verfahren zum Herstellen eines Acrylgelmaterials durch Vernetzen einer Zusammensetzung, die ein Acrylatpolymer und einen mit dem Acrylatpolymer verträglichen, weichmachenden, flüssigen Bestandteil umfaßt, unter Bilden einer vernetzten Gelschicht, mit der eine Oberfläche eines Substrats versehen ist, wobei das Gewichtsverhältnis des Acrylatpolymers zu dem weichmachenden, flüssigen Bestandteil 1,0/0,25 bis 1,0/2,0 beträgt und die Dicke der vernetzten Gelschicht 40 bis 150 µm beträgt, mit der Maßgabe, daß die vernetzte Gelschicht keine Doppelschicht ist und daß das Substrat kein durchlässiges Substrat ist.

2. Verfahren von Anspruch 1, bei dem das Gewichtsverhältnis des Acrylatpolymers zu dem weichmachenden, flüssigen Bestandteil 1,0/0,4 bis 1,0/2,0 beträgt.

3. Verfahren von Anspruch 2, bei dem das Gewichtsverhältnis des Acrylatpolymers zu dem weichmachenden, flüssigen Bestandteil 1,0/0,6 bis 1,0/1,8 beträgt.

4. Verfahren von Anspruch 1, bei dem die Zusammensetzung durch Verwenden eines Vernetzungsmittels vernetzt wird, das aus einem Metallalkoholat und einem Metallchelat, die jeweils Titan oder Aluminium umfassen, und einem trifunktionellen Isocyanat ausgewählt ist.

5. Verfahren zum Herstellen einer Acrylgelzubereitung durch Zufügen eines Arzneimittelbestandteils zu dem in Anspruch 1 beanspruchten Acrylgelmaterial.

6. Verfahren von Anspruch 5, bei dem der Gehalt des Arzneimittelbestandteils von 1 bis 40 Gew.-% bezogen auf die Gesamtmenge der vernetzten Gelschicht beträgt.

7. Verfahren von Anspruch 6, bei dem der Gehalt des Arzneimittelbestandteils von 3 bis 30 Gew.-% bezogen auf die Gesamtmenge der vernetzten Gelschicht beträgt.

## Revendications (Revendications pour l'(les) Etat(s) contractant(s) suivant(s): CH, DE, FR, GB, IT, LI, NL.)

1. Matériel de gel acrylique comprenant un substrat présentant sur une de ses surfaces une couche de gel réticulé formée par réticulation d'une composition comprenant un polymère d'acrylate et un constituant liquide plastifiant compatible avec ledit polymère d'acrylate, dans lequel le rapport pondéral dudit polymère d'acrylate audit constituant liquide plastifiant est compris entre 1,0/0,25 et 1,0/2,0 et l'épaisseur de ladite couche de gel réticulé est comprise entre 40 et 150 µm, pour autant que la couche de gel réticulé n'est pas une couche double et que le substrat n'est pas un substrat perméable.

2. Matériel de gel acrylique selon la revendication 1, dans lequel le rapport pondéral dudit polymère d'acrylate audit constituant liquide plastifiant est compris entre 1,0/0,4 et 1,0/2,0.

3. Matériel de gel acrylique selon la revendication 2, dans lequel le rapport pondéral dudit polymère d'acrylate audit constituant liquide plastifiant est compris entre 1,0/0,6 et 1,0/1,8.

4. Matériel de gel acrylique selon la revendication 1, dans lequel la réticulation s'effectue par emploi d'un agent réticulant sélectionné parmi un alcoolate de métal et un chélate de métal comprenant chacun du titane ou de l'aluminium et un isocyanate trifonctionnel.

5. Préparation de gel acrylique dans laquelle un composant médicamenteux est contenu dans un matériel de gel acrylique selon la revendication 1.

6. Préparation de gel acrylique selon la revendication 5, dans laquelle la teneur en dit composant médicamenteux est comprise entre 1 et 40 % en poids par rapport à la quantité totale de ladite couche de gel réticulé.

7. Préparation de gel acrylique selon la revendication 6, dans laquelle la teneur en dit composant médicamenteux est comprise entre 3 et 30 % en poids par rapport à la quantité totale de ladite couche de gel réticulé.

## Revendications (Revendications pour l'(les) Etat(s) contractant(s) suivant(s): ES)

1. Procédé de préparation d'un matériel de gel acrylique par réticulation d'une composition comprenant un polymère d'acrylate et un constituant liquide plastifiant compatible avec ledit polymère d'acrylate pour former une couche de gel réticulé située sur une des surfaces d'un substrat, dans lequel le rapport pondéral dudit polymère d'acrylate audit constituant liquide plastifiant est compris entre 1,0/0,25 et 1,0/2,0 et l'épaisseur de ladite couche de gel réticulé est comprise entre 40 et 150 µm, pour autant que la couche de gel réticulé n'est pas une couche double et que le substrat n'est pas un substrat perméable.

2. Procédé selon la revendication 1, dans lequel le rapport pondéral dudit polymère d'acrylate audit constituant liquide plastifiant est compris entre 1,0/0,4 et 1,0/2,0.

3. Procédé selon la revendication 2, dans lequel le rapport pondéral dudit polymère d'acrylate audit constituant liquide plastifiant est compris entre 1,0/0,6 et 1,0/1,8.

4. Procédé selon la revendication 1, dans lequel ladite composition est réticulée par emploi d'un agent réticulant sélectionné parmi un alcoolate de métal et un chélate de métal comprenant chacun du titane ou de l'aluminium et un isocyanate trifonctionnel.

5. Procédé de préparation d'une préparation de gel acrylique par addition d'un composant médicamenteux au matériel de gel acrylique selon la revendication 1.

6. Procédé selon la revendication 5, dans lequel la teneur en dit composant médicamenteux est comprise entre 1 et 40 % en poids par rapport à la quantité totale de ladite couche de gel réticulé.

7. Procédé selon la revendication 6, dans lequel la teneur en dit composant médicamenteux est comprise entre 3 et 30 % en poids par rapport à la quantité totale de ladite couche de gel réticulé.
